# EUROPEAN PATENT APPLICATION

(11) **EP 2 261 542 A1**
(43) Date of publication of application: **15.12.2010**
(21) Application number: 10380076.9
(22) Date of filing: 02.06.2010
(51) Int. Cl.: F16L 11/08

(54) **Pipe for water-supply systems**

(30) Priority: 03.06.2009 ES 200930257
(71) Applicant: ABN Pipe Systems, S.L.U., 15008 A Coruña (ES)
(72) Inventor: Vázquez Sánchez, Javier Antonio, 15008 - A Coruña (ES)
(74) Representative: Carvajal y Urquijo, Isabel

(57) **Abstract**

A pipe for water-supply systems of the type made from plastic material, that comprises at least, an external layer (1) and an internal layer (2) both made of a material selected from a thermoplastic material, a thermostable material or a combination of both, the internal wall (2) of which, in its most internal wall, which remains in contact with water, comprises an antimicrobial and/or antibacterial additive, being said additive integrated into the internal layer by means of co-extrusion.

## Description

### FIELD AND OBJECT OF THE INVENTION

The invention relates to the field of cold and hot sanitary water piping system and more specifically to a multilayer pipe that has the characteristic of comprising an internal antibacterial and/or antimicrobial treatment.

### STATE OF THE ART

Nowadays there are known multiple types of pipes for water supply, in most cases made of plastic materials, due to their strength and low cost in comparison to metal pipes.

Normally, the internal wall of the pipes or conduits is the one that is in direct contact with the liquid to be conveyed. Bacteria (legionella, among others), fungi, yeast, seaweed, and other microorganisms which may be contained in the water itself and which may be developed in said internal wall adhere to the aforementioned wall, being capable of forming strains or plaques, which, on the one hand, damage the quality and sanitary conditions of water of the liquid conveyed and on the other hand collaborate in a very active way on the deterioration of the pipe itself reaching in some occasions the partial or total destruction thereof.

On the other hand, it is known that the thermoplastic tubes have certain amount of permeability to liquids, gases and smells. This amount varies according to the plastic raw material used. Some raw materials are more permeable to liquids and by contrast its behavior towards the permeability of gases is better and vice versa. In fact, there exist tube developments with oxygen barriers which are used for heating systems and where those barriers are made of a layer of EVOH, but in said tubes a completely effective barrier against liquids and smells is not achieved.

Likewise there exist plastic tubes that have an intermediate aluminum layer that serves as a gas and liquid barrier and which is used in installations in contaminated terrains such as refineries, however, this is not a perfect barrier because in order to join the tubes it is necessary to eliminate part of it to be able to weld it and therefore the tube has a discontinued impermeability and as the plastic material is joined to the aluminum by means of an adhesive layer the junction is not perfect.

If we analyze where the pipes for water conveyance in the water supply system are placed, we observe that they are installed in all kinds of terrains which in many cases may be contaminated. Besides, these systems circulate parallel to the sewage treatment systems, which are not usually impermeable, because pipes such as concrete pipes are used which are not impermeable themselves and which also have many non-watertight junctions, whereby these zones become contaminated due to leakage from these tubes and pipes from the water-supply system run across these zones.

For all the reasons mentioned above, it has been detected a need for providing a pipe made of some base material of plastic nature, to which an antibacterial and/or antimicrobial additive is added, as well as a layer that serves as a barrier against the passage of either liquid or gaseous fluids and against smells.

This objective is achieved by means of the invention just as it is defined in claim 1; in the depending claims preferred embodiments of the invention are defined.

### DESCRIPTION OF THE INVENTION

The present invention relates to a pipe for water distribution networks of the type made from plastic material, which comprises at least an external layer and an internal layer, both made from a thermoplastic material, a thermostable material or a combination of both, the internal layer of which, in its most internal wall, which remains in contact with water, comprises an antimicrobial and/or antibacterial additive, being said additive integrated into the internal layer by means of co-extrusion.

In this way, it is provided a permanent protection against the degradation caused by bacteria, mold and fungi extending the service life of pipes and avoiding the microorganisms which may cause deterioration and bad smell of water, guaranteeing in this way the quality thereof. On the other hand, the pipes object of the present invention, have excellent internal abrasion strength thanks to their layer of polyethylene, polypropylene and other additive enhanced materials (blue color) and consequently maintain the internal evenness during their entire service life. For this reason, it is possible to obtain a greater flow capacity and minimum friction load losses.

The external and internal layers may be made from a material selected from polypropylene (PP), reinforced high density polyethylene (HDPE), Acrylonitrile butadiene styrene (ABS), Polyamides, Polyvinyl chloride (PVC) or any combination thereof.

Advantageously, the internal layer may be made from polyamide due to the optimal superficial quality characteristics of this material that keep the internal roughness coefficient to a minimum. This greater internal evenness and the decrease of load losses help to have less scale formations in the interior of the tube due to roughness decrease.

With regard to pipe thickness, the thickness of the internal layer E_{CI} may be between 1 and 25% of the total thickness E_{T} of the pipe, being the optimal value of thickness of the internal layer E_{CI} within 2 and 15% of the total thickness E_{T} of the pipe. In the total thickness of the pipe these percentages usually represent from 0.3 to 0.6 mm of the pipe thickness so that the antimicrobial agent has the adequate effectiveness. These value ranges are not arbitrary, but there exists an adequate configuration for the manufacturing of multilayer tubes depending on the tube diameter which is being manufactured, presenting substantial differences such as a 12mm diameter or a 630 mm diameter.

In a second aspect of the invention it is provided the possibility of adding an intermediate layer placed between the internal and the external layer, which is impermeable to water, gases and smell and which may be made from a thermoplastic material, a thermostable material, or a combination of both, a polyamide; EVOH and nanoclays. The proportion of nanoclays will be between 3 and 10% by weight of the total composition of the intermediate layer and between 7 and 15% by weight of EVOH. The optimal proportions of these components are 5% by weight of nanoclays and 10% by weight of EVOH.

The thickness of the intermediate layer E_{CINT} will be between 10 and 40% of the total thickness E_{T} of the piping, being 15% the optimal percentage.

With regard to the antimicrobial and/or antibacterial additive that will be possibly used, internally covering the internal layer there will be salts of silver, triclosan or BIOSAFE ® on silane base, or a combination thereof. This choice was obtained by the manufacturing test of different materials and the subsequent assay of the bactericidal effect that presented the tubes with different internal layer thickness. The tests were not carried out on ordinary drinking water, but instead to show the action of the bactericidal additives different microorganisms were inoculated (yeast, bacteria and fungi) into the water contained in the tube. In this way, it could be observed the true effect that the antimicrobial additive has on each microorganism as well as the appropriate configuration of the layers for their correct and maximum effectiveness.

In other tests, real installations of the tube with the antimicrobial agent were conducted for an estimated time greater than 4000H in problematic environments for generation of deposits and calcifications, finding that in the tube without the antimicrobial agent the flow of the tube has been completely stopped, while in the tube designed with the bactericidal additive no remarkable deposit has been produced. These aspects remark again the greater flow, the internal evenness, the lesser friction losses, the absence of deposit development and the effective antimicrobial action.

Other series of advantageous characteristics of the piping is that it has great stability outdoors thanks to its great insulating capacity, its low thermal conductivity and its flexibility, being protected against the degradation caused by ultraviolet rays, being able to be stored or installed in the open for long periods without any damage or loss of its physical and mechanical properties. Additionally, its great flexibility allows it to bend and absorb impact loads. The tensile breaking strength in the terrain is very high, thanks to a novel manufacturing process, ensuring that there will not be any problem in the installation service if superficial scratches are produced equivalent to a length of 1/10 of the piping thickness. Pipes weight considerably less than all other types of pipes that are usually used in pressurized water facilities, making its handling and installation easier, obtaining important savings on labor and machinery.

It is worth noticing that the pipes are chemically inert, so the aggressive products of the ground cannot attack them or cause any type of degradation thereto. The HDPE, for example, is not an electrical conductor, so it is not affected by oxidation or corrosion by electrolytic action, as cast-iron and steel pipes are, which for that reason may break down in a very short time. On the other hand, the antimicrobial additive will remain perfectly joined to the polymer and it has been tested in assays of migration to drinking water, so that it will be proven that none of the additives added for improving the antimicrobial characteristics of the material may by no means, in any case, reach the drinking water and contaminate it. It has been proven that there exist no concentrations of any contaminating element in the drinking water by means of assays under the current national and European legislation.

The pipe object of the present invention incorporates antimicrobial additives which do not damage in any case the product usage requirements and which also contribute to the improvement of the mechanical, thermal and pressure properties in relation to standard tubes without additives.

### DESCRIPTION OF THE DRAWINGS

There follows a brief description of a series of drawings which help understand the invention better particularly relating to some embodiments of said invention presented as illustrative and non-limiting examples thereof.
Figure 1 represents a sectional view according to a cutting plane perpendicular to the axis of a first preferred embodiment of the pipe object of the present invention.
Figure 2 represents a perspective view of a small portion of the pipe corresponding to Figure 1.
Figure 3 represents a sectional view according to a cutting plane perpendicular to the axis of a second preferred embodiment of the pipe object of the present invention.
Figure 4 represents a perspective view of a small portion of the pipe in which approximately a quarter thereof, corresponding to the pipe of Figure 3, has been eliminated.
Figure 5 represents a sectional view according to a longitudinal cutting plane of a portion of the pipe object of the present invention.

### DESCRIPTION OF A PREFERRED EMBODIMENT OF THE INVENTION

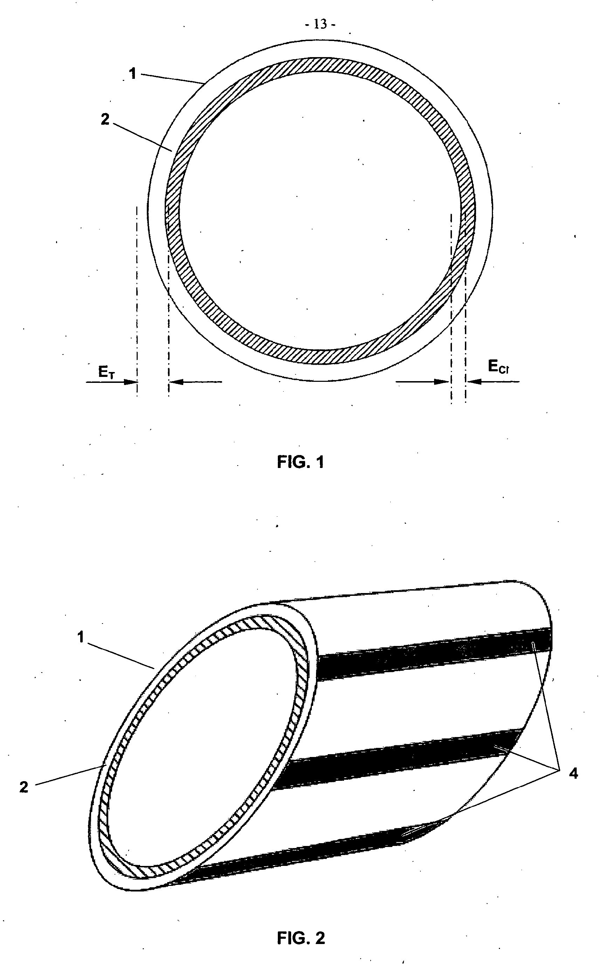

As it may be seen in Figures 1 and 2, the pipe object of the present invention, in its first preferred embodiment, consists of an external layer (1) that is made of polypropylene (PP), reinforced high density polyethylene (HDPE), Acrylonitrile butadiene styrene (ABS), Polyamides, Polyvinyl chloride (PVC) or a combination thereof and concentrically, the pipe has an internal layer (2) which can be made of the same materials. Suitable for the internal layer (1) but to which an antimicrobial and/or antibacterial additive has been added in the most internal wall of said internal layer (2) which is in direct contact with water. The integration of the additive into the internal layer (2) is carried out during the shaping of the pipe by means of a co-extrusion procedure. The additive used will depend on the material that the internal layer (2) is made of, whether it is polyethylene, polypropylene or any of the aforementioned polymers, being it possible to choose as antimicrobial additives salts of silver, triclosan, or BIOSAFE ® on silane base, provided that the compatibility of both materials is appropriate, so that adhesion is optimal for achieving effectiveness and without facilitating migration.

As it can be seen in Figure 1, the pipe has a total thickness. E_{T} and the internal layer has a thickness E_{CI} being E_{CI} between 1 and 25% of E_{T}, being the optimal percentages in a range between 2 and 15.

The external layer (1) has high impact strength and exceptional crack growth strength, more than 100 times.the parameters required by the European standards for drinking water and gas polyethylene. It is highly resistant to UV rays, being it possible to make outdoor installations without any type of precautions or coverings. Additionally, it has external bands (4), longitudinally placed and parallel between them that have a color different to the color of the external layer (1) so that they can be distinguished from it and this type of pipes may perfectly identify.

The internal layer (2) provides high strength to internal pressure and wear and it has a practically null friction coefficient.

The pipes could be used for the supply of water systems, in aerial as well as buried installations.

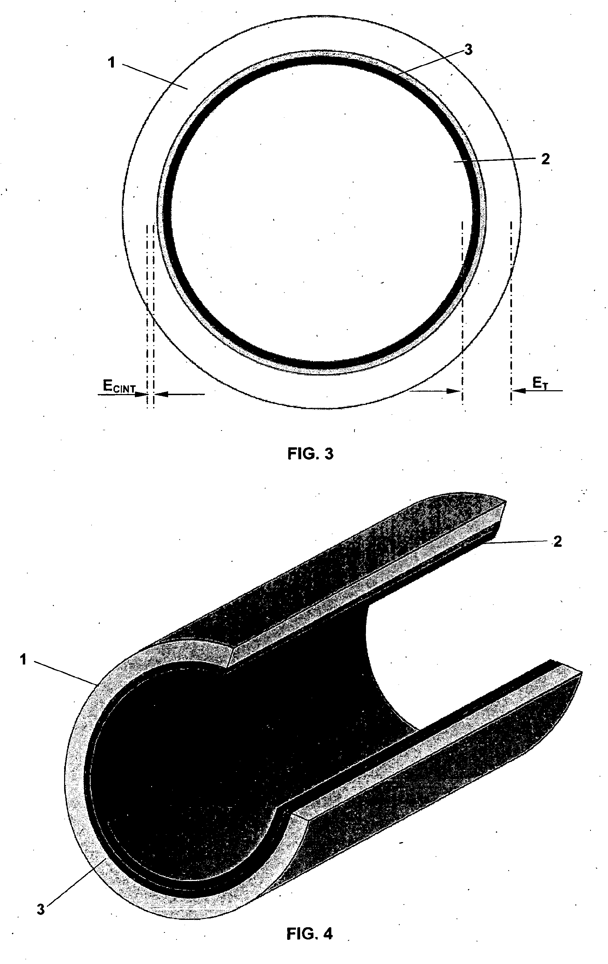

In Figures 3 and 4, a second preferred embodiment of the invention has been represented, in which, as well as having the internal layer (2) and the external layer (1) it has between them a third intermediate layer (3) which is impermeable to the passage of liquid and gaseous fluids and smells. The material constituting intermediate layer (3) will be a combination of a thermoplastic or thermostable material or a combination of both which will be the base of said layer and also as impermeable elements, a polyamide, EVOH and nanoclays. The percentage by weight on the total composition in the case of the nanoclays will be between 1 and 10%, being 2,5% the ideal percentage. As regards EVOH, the percentage by weight on the total composition will be between 2 and 10%, being 3% the optimal value.

As regards the relation between the thickness E_{CINT} of the intermediate layer (3) and the total thickness E_{T}, it will be between 5 and 40%, being 10% the optimal relation.

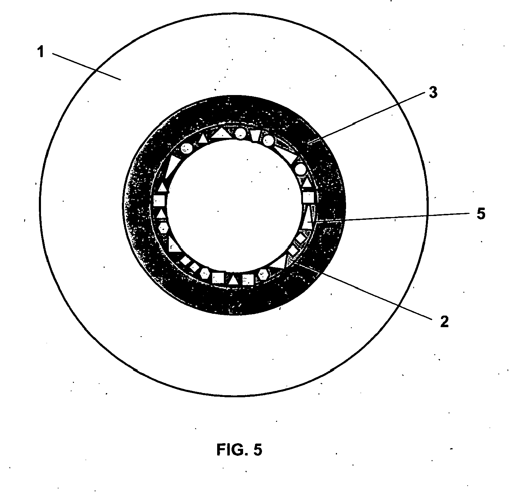

In Figure 5, it has been represented a section of a pipe portion according to the present invention, in which it may be observed the distribution of the antimicrobial additives, schematically represented by means of geometrical forms (5), in relation to the internal wall of the pipe which is in direct contact with water, as it can be seen, the distribution of the additives is such that they have to stay placed as close as possible to the water layer, being the distribution as even as possible so that the contact surface between the antimicrobial additive and water be increased to the maximum.

There follows an attached summary table with the purpose of proving the advantages of the pipe object of the present invention, which contains assays related to the growth of microorganisms inoculated at 96 hours, with microorganism life cycle without food and at a temperature of 30°C.

| | Tube WITH optimized and antimicrobial thickness | Tube WITHOUT optimized and antimicrobial thickness | Standard tube without antimicrobial |
|---|---|---|---|
| Microorganism 1 (aerobic mesophiles) | / 2570 | X 2300 | X 684000 |
| Microorganism 2 (yeast) | /5 | X 2 | X 2 |
| Microorganism 3 (fecal coliforms) | / 500 | X 3000 | X 840000 |
| Microorganism 4 (legionella) | / 3 | X 6 | X 10 |

For interpreting the data contained in the table the following facts have to be taken into account:
(1) For determining the data of microbial increase or decrease ufc/ml has been used as a measure unit representing the colony formation units of the microorganism to be evaluated per milliliter of analyzed water. In microbiology, it is customary that the recount is carried out by colonies or "microorganism groupings", instead of counting individual microorganisms.
(2) In the table it is represented by "/factor" the percentage of microorganism disappearance. Y by "x factor": the percentage of microorganism multiplication.
(3) The different microorganisms are listed; some of them grow more easily than the others, which is why there are such differences in the size orders.

As it can be seen from the data shown in the table, it is evident that if the antimicrobial is added, but the appropriate layer thickness is not added, none of the analyzed microorganisms die, rather their growth and multiplication is favored. Therefore, the antimicrobial layer thickness is a determining factor.

On the other hand, there follows a table of migration assays according to the standard ISO 8795 in which measures of the content of additive in water and in the tube are shown for a tube with antimicrobial additive optimized thickness, taking measures for periods of 24 hours, 168 hours, 2000 hours and 4300 hours, respectively. Additionally, the limit permitted by the legislation of additive content for the aforementioned periods were included.

| | Tube WITH optimized and antimicrobial thickness Period = 24 h | Tube WITH optimized and antimicrobial thickness Period = 168 h | Tube WITH optimized and antimicrobial thickness Period = 2000 h | Tube WITH optimized and antimicrobial thickness Period = 4300 h |
|---|---|---|---|---|
| Content of Additive in Water | <0,002 mg/l | <0,002 mg/l | <0,002 mg/l | <0,002 mg/l |
| Content of Additive in Tube | < 1 g/kg | < 1 g/kg | < 1 g/kg | < 1 g/kg |
| Limit permitted by the Legislation | <0,010 mg/l | <0,010 mg/l | <0,010 mg/l | <0,010 mg/l |

As it can be seen from the data shown in the table, the concentration of additive in water is five times smaller than the value permitted by the legislation and the tube neither loses additive with time nor does its effectiveness disappear, instead, the additive concentration in the tube is constant. Therefore, by using the antimicrobial additives mentioned in the present invention, according to an optimized thickness of the additive layer, it is achieved that the migration of said additive to the water conveyed by the pipe be such that there is no water contamination and therefore the pipe object of the present invention is safe for conveying water for human uses.

## Claims

1. Pipe for water-supply systems of the type made of plastic material, **characterized in that** it comprises at least an external layer (1) and an internal layer (2) both made of a material selected from a thermoplastic material, a thermostable material or a combination of both, the internal wall (2) of which, in its most internal wall, remains in contact with water, comprises an antimicrobial and/or antibacterial additive.

2. Pipe according to claim 1, **characterized in that** the external layer (1) and the base of the internal layer (2) are made of a material selected from polypropylene (PP), reinforced high density polyethylene (HDPE), Acrylonitrile butadiene styrene (ABS), Polyamides, Polyvinyl chloride (PVC) or a combination thereof.

3. Pipe according to claim 2, **characterized in that** the internal layer (2) is made of polyamide.

4. Pipe according to the preceding claims, **characterized in that** the internal layer (2) thickness E_{CI} is between 1 and 25% of the total thickness E_{T} of the pipe.

5. Pipe according to claim 4, **characterized in that** the internal layer (2) thickness E_{CI} is between 2 and 15 % of the total thickness E_{T} of the pipe.

6. Pipe according to the preceding claims, **characterized in that** between the external layer (1) and the internal layer (2) there is an additional intermediate layer (3) impermeable to water, gases and smells comprising a material selected from a thermoplastic material, a thermostable material or a combination of both, a polyamide, EVOH and nanoclays.

7. Pipe according to claim 6, **characterized in that** the intermediate layer (3) comprises between 1 and 10% by weight of nanoclays and between 2 and 10% by weight of EVOH.

8. Pipe according to claim 7, **characterized in that** the intermediate layer (3) comprises between 2,5% by weight of nanoclays and between 10% by weight of EVOH.

9. Pipe according to claims 6 to 8, **characterized in that** the intermediate layer (3) thickness E_{CINT} is between 5 and 40 % of the thickness E_{T} of the pipe.

10. Pipe according to claim 9, **characterized in that** the intermediate layer (3) thickness E_{CINT} is 10% of the thickness E_{T} of the pipe.

11. Pipe according to the preceding claims, **characterized in that** the antimicrobial and/or antibacterial additive of the internal layer (2) is selected from salts of silver, triclosan or BIOSAFE ® on silane base, or a combination thereof.
